# EUROPÄISCHE PATENTSCHRIFT

(11) **EP 2 513 009 B1**
(45) Veröffentlichungstag und Bekanntmachung des Hinweises auf die Patenterteilung: **22.03.2017**
(21) Anmeldenummer: 10795340.8
(22) Anmeldetag: 16.12.2010
(51) Int. Cl.: A61L 27/04, C04B 35/645, A61L 31/02, C04B 35/119

(54) **KERAMISCHER VERBUNDWERKSTOFF, BESTEHEND AUS DEN HAUPTBESTANDTEILEN ALUMINIUMOXID UND ZIRKONOXID**
CERAMIC COMPOSITE MATERIAL COMPRISING ALUMINA AND ZIRCONIA
MATERIAU CERAMIQUE COMPOSITE CONTENANT D' ALUMINE ET DE ZIRCONE

(30) Priorität: 16.12.2009 DE 102009054797; 16.12.2009 DE 102009054796
(43) Veröffentlichungstag der Anmeldung: 24.10.2012
(73) Patentinhaber: CeramTec GmbH, 73207 Plochingen (DE)
(72) Erfinder: KUNTZ, Meinhard, 73733 Esslingen (DE); KUNTZ, Michael, 66424 Homburg (DE); GOTTWIK, Lukas, 73092 Heiningen (DE); SCHILCHER, Kristina, 73730 Esslingen (DE); MORHARDT, Andreas, 73734 Esslingen (DE); FRIEDERICH, Kilian, 73207 Plochingen (DE); SCHNEIDER, Norbert, 73614 Schorndorf (DE)
(74) Vertreter: Uppena, Franz
(86) Internationale Anmeldenummer: PCT/EP2010/069991
(87) Internationale Veröffentlichungsnummer: WO 2011/083022

(56) Entgegenhaltungen:
- EP-A1- 2 168 936
- WO-A2-01/80783
- US-A- 5 032 555
- US-A1- 2002 010 070
- "Zirconiumoxid" In: W. Kollenberg (Hrsg.): "Technische Keramik; Grundlagen, Werkstoffe, Verfahrenstechnik; 2. Auflage", 30. November 2009 (2009-11-30), VULKAN VERLAG, D- 45128 Essen, XP002629667, ISBN: 978-3-8027-2927-7 Seiten 231-250, Seite 234, Bild 69 (c); und erster folgender Absatz; Seite 240-->"3.2.3.4" bis Seite 242, Ende des ersten Absatzes; Seite 243-->"3.2.3.6" bis Ende von Seite 245; Seite 247-->"Medizintechnik"; Seiten 248-250; Literaturstellen (1), (15), (64) und (65).
- HANNINK R H J: "TRANSFORMATION TOUGHENING IN ZICONIA-CONTAINING CERAMICS", JOURNAL OF THE AMERICAN CERAMIC SOCIETY, BLACKWELL PUBLISHING, MALDEN, MA, US, Bd. 83, Nr. 3, 1. Januar 2000 (2000-01-01) , Seiten 461-487, XP008078755, ISSN: 0002-7820

## Beschreibung

Die vorliegende Erfindung betrifft einen aus Aluminiumoxid als keramische Matrix und darin dispergiertem Zirkonoxid bestehenden Verbundwerkstoff, ein Verfahren zu dessen Herstellung und dessen Verwendung.

Die molekularen Strukturen von metallischen Legierungen und keramischen Werkstoffen unterscheiden sich wesentlich. In der Metallbindung kreisen die Elektronen ungeordnet und mit vergleichsweise geringer Bindungskraft um die Atomkerne. Aus diesem "lockeren" Gefüge lösen sich, beispielsweise im Körpermilieu, ständig Ionen; vielfältige chemische Reaktionen sind möglich.

In keramischen Molekülen folgen die Elektronen in der Keramikbindung exakt vorgegebenen Bahnen, den sogenannten gerichteten Elektronenorbitalen. Ihre Bindungskraft ist sehr hoch, die Moleküle sind äußerst stabil. Deshalb kommt es nicht zur Bildung von Ionen, und chemische Reaktionen sind praktisch ausgeschlossen.

Die extrem stabile Keramikbindung schließt eine plastische Verformung des Materials nahezu aus. Dies bewirkt einerseits die gewünschte extrem hohe Härte, führt jedoch auf der anderen Seite zu einer relativ hohen Sprödheit. Mit dem richtigen Werkstoffdesign kann man jedoch gleichzeitig eine hohe Härte und eine hohe Zähigkeit erreichen.

Die Materialwissenschaft unterscheidet zwischen Bruchfestigkeit und Bruchzähigkeit. Die Bruchfestigkeit bezeichnet die maximale mechanische Spannung, die ein Material aushält, ohne zu brechen. Bruchzähigkeit, oder auch Risszähigkeit, beschreibt den Widerstand eines Materials gegen einsetzendes Risswachstum. In der Medizintechnik werden bereits heute keramische Materialien eingesetzt, die eine sehr hohe Bruchfestigkeit aufweisen. Einige dieser keramischen Materialien sind zusätzlich mit einer extrem hohen Bruchzähigkeit ausgestattet. Solche Materialien können viel besser als andere Keramiken einsetzenden Rissen widerstehen und einen Rissverlauf unterbrechen.

Diese Eigenschaft beruht auf zwei Verstärkungsmechanismen. Der erste Verstärkungsmechanismus ist den eingelagerten tetragonalen Zirkonoxid-Nanopartikeln zu verdanken. Diese Partikel sind einzeln in der stabilen Aluminiumoxid-Matrix verteilt. Sie erzeugen lokale Druckspitzen im Bereich der Risse und wirken so gegen die Rissausbreitung.

Der zweite Verstärkungsmechanismus wird durch plättchenförmige Kristalle erreicht, die sich in der Oxidmischung ebenfalls vereinzelt bilden. Diese "Platelets" lenken mögliche Risse um, zerstreuen Rissenergie und bauen sie damit ab. Beide Funktionen erlauben es, mit solchen Materialien auch Komponentengeometrien zu konstruieren, die früher mit Keramik nicht zu erreichen waren.

Dabei ist es von Vorteil, wenn das Zirkonoxid im keramischen Werkstoff in der tetragonalen Phase vorliegt. Es ist diesbezüglich allgemein bekannt, dass eine solche Stabilisierung ab einer gewissen Menge von Zirkonoxid im Werkstoff nur durch die Zugabe von stabiliserenden Oxide (chemische Stabiliserung möglich ist ("Zirconiumoxid" in: W. Kollenberg (Hrsg): "Technische Keramik; Grundlagen, Werkstoffe, Verfahrenstechnik"; 2. Auflage", 30. November 2009 (2009-11-30)' VULKAN VERLAG).

Hannink et al. offenbaren Zirkonoxid-stabilisierte Aluminiumoxidkeramiken (ZTA) und verweisen ebenfalls auf die wichtige tetragonale Phase des Zirkoniumoxids. Bei den verschiedenen Möglichkeiten, die tetragonale Phase zu stabiliseren offenbaren Hannink et al. hauptäschlich die Zugabe von stabilisierenden Oxiden wie Yttriumoxid oder Ceroxid (Richard H. Hannink: J. Am. Ceram. Soc.,83 (3) 461-87 (2000).

Die EP 2 168 936 A1 beispielsweise offenbart ein Verfahren zur Herstellung eines feinteiligen Werkstoffes, welcher aus Aluminiumoxid und Zirkoniumoxid besteht, wobei das Zirkoniumoxid in der tetragonalen Phase vorliegen kann. Eine entsprechende Stabiliserung erfolgt nach dieser Druckschrift über eine gewisse Menge an Stabilisatoroxiden wie Magnesiumoxid oder Ceroxid.

In der US 2002/010070 A1 wird ebenfalls eine ZTA-Keramik beschrieben, die beispielsweise in medizintechnischen Anwendungen, wie der Implanttechnik verwendet werden kann. Die Stabilisierung der tetragonalen Phase des Zirkoniumoxids erfolgt dabei über die Zugabe von mindestens 2,5 Mol.% Yttriumoxid.

Ein Verfahren zur Herstellung einer ZTA-Keramik wird außerdem in der US 5,032,55 A offenbart. Auch hierbei wird tetragonal stabilisiertes Zirkoniumoxid eingesetzt, wobei die Stabilisierung durch Zugabe von mindestens 1 Gew.-% Yttriumoxid erreicht wird.

Die der vorliegenden Erfindung zugrundeliegende Aufgabe bestand darin, die Eigenschaften der bekannten keramischen Materialien weiter zu verbessern.

Die vorliegende Erfindung betrifft einen keramischen Verbundwerkstoff, bestehend aus den Hauptbestandteilen Aluminiumoxid und Zirkonoxid, sowie einem oder mehreren anorganischen Zuschlagstoffen, mit denen die Eigenschaften des Verbundwerkstoffs beeinflusst werden können. Dabei bildet Aluminiumoxid die Hauptkomponente mit einem Volumengehalt von > 65 %, vorzugsweise 85 bis 90 %, das Zirkonoxid bildet die Nebenkomponente mit einem Volumengehalt zwischen 10 und 35 %. Sowohl Aluminiumoxid als auch Zirkonoxid können weiterhin lösliche Bestandteile enthalten. Als lösliche Bestandteile können ein oder mehrere der folgenden Elemente vorliegen: Cr, Fe, Mg, Ti, Y, Ce, Ca, Lanthanide und/oder V. Das Zirkonoxid liegt im Ausgangszustand zu einem überwiegenden Teil, vorzugsweise zu 80 bis 99 %, besonders bevorzugt von 90 bis 99 %, bezogen auf den Geamtzirkonoxidgehalt, Ausgangszustand zu einem überwiegenden Teil, vorzugsweise zu 80 bis 99 %, besonders bevorzugt von 90 bis 99 %, bezogen auf den Geamtzirkonoxidgehalt, in der tetragonalen Phase vor. Die bekannte Phasenumwandlung des Zirkonoxids von tetragonal zu monoklin wird bei dem erfindungsgemäßen Verbundwerkstoff als Verstärkungsmechanismus genutzt, um die Risszähigkeit und die Festigkeit günstig zu beeinflussen.

Die Stabilisierung der tetragonalen Phase des Zirkonoxids erfolgt im erfindungsgemäßen Verbundwerkstoff zum überwiegenden Teil überraschenderweise nicht chemisch sondern mechanisch. Daher ist der Gehalt an anorganischen chemischen Stabilisatoren relativ zum Zirkonoxid auf Werte begrenzt, die deutlich unterhalb der im Stand der Technik normalerweise verwendeten Gehalte liegen. Der im Stand der Technik üblicherweise bevorzugt verwendete chemische Stabilisator ist Y₂O₃. Weitere bekannte Stabilisatoren sind CeO₂, CaO und MgO.

Beispiele bekannter Rezepturen für keramische Verbundwerkstoffe sind:

| **Bezeichnung** | **Mol % Y₂O₃ bezogen auf ZrO₂** |
|---|---|
| Y-TZP⁽¹⁾ | 2,8 oder 3,2 |
| ZTA⁽²⁾ | 1,3 |

| | |
|---|---|
| ⁽¹⁾ Yttrium toughened Zirconia ⁽²⁾ Zirconia toughened Alumina | |

Im erfindungsgemäßen Verbundwerkstoff wird ein Stabilisatorgehalt verwendet, der deutlich niedriger ist, als die im Stand der Technik verwendeten Gehalte. Erfindungsgemäß wird dies dadurch ermöglicht, dass in dem erfindungsgemäßen Verbundwerkstoff das Zirkonoxid derart in die Aluminiumoxid-Matrix eingebettet wird, dass es durch die Einbettung in der Matrix in der metastabilen tetragonalen Phase stabilisiert wird (mechanische Stabilisierung).

Voraussetzung für die mechanische Stabilisierung ist ein Aluminiumoxidanteil von mindestens 65 Vol.-%, vorzugsweise von 65 bis 90 Vol.-%, bei einem Zirkonoxidanteil von 10 bis 35 Vol.-%. Von besonderer Bedeutung für die erfindungsgemäß überraschenderweise erzielbare mechanische Stabilisierung ist die Korngröße der Zirkonoxidpartikel im erfindungsgemäßen Verbundwerkstoff. Die Korngröße der Zirkonoxidpartikel sollte durchschnittlich 0,5 µm nicht übersteigen (gemessen nach Linienschnittverfahren). Bevorzugt für den erfindungsgemäß mechanisch stabilisierten Verbundwerkstoff sind Zirkonoxidpartikel einer Korngröße von durchschnittlich 0,1 µm bis 0,2 µm, 0,2 µm bis 0,3 µm, 0,3 µm bis 0,4 µm oder von 0,4 µm bis 0,5 µm, bevorzugt von 0,1 µm bis 0,3 µm, besonders bevorzugt von 0,15 µm bis 0,25 µm.

Der Anteil an chemischen Stabilisatoren im erfindungsgemäßen Verbundwerkstoff (Anteil jeweils relativ zum Zirkonoxidgehalt) beträgt für Y₂O₃ ≤ 1,5 Mol%, bevorzugt ≤ 1,3 Mol%, für CeO₂≤ 3 Mol%, für MgO ≤ 3 Mol% und für CaO ≤ 3 Mol%. Besonders bevorzugt ist ein Gesamtgehalt an Stabilisatoren von weniger als 0,2 Mol%. Erfindungsgemäß ganz besonders bevorzugt ist ein mechanisch stabilisierter Verbundwerkstoff der keinen chemischen Stabilisator enthält.

Es ist bekannt, dass Werkstoffe, die durch die Verwendung von chemischen Stabilisatoren, insbesondere Werkstoffe, die durch Y₂O₃ stabilisiert sind, zu hydrothermaler Alterung neigen. Bei diesen Werkstoffen tritt eine spontane Phasenumwandlung in Anwesenheit von Wassermolekülen bei erhöhten Temperaturen, beispielsweise bereits bei Körpertemperatur auf. Die Ursache für diese Empfindlichkeit gegenüber Wasser bei erhöhten Temperaturen ist die Ausbildung von Sauerstoffleerstellen im Zirkonoxid-Gitter, die von Hydroxidionen besetzt werden können. Dieses Phänomen wird "hydrothermale Alterung" genannt.

Der erfindungsgemäße Verbundwerkstoff weist eine deutlich geringere Neigung zu hydrothermaler Alterung auf, als Werkstoffe, die durch die Verwendung von chemischen Stabilisatoren, insbesondere durch die Verwendung von Y₂O₃ stabilisiert sind.

Durch den reduzierten Gehalt an chemischen Stabilisatoren enthält das Zirkonoxidgitter in dem erfindungsgemäßen Verbundwerkstoff proportional weniger Sauerstoffleerstellen. Somit reagiert der erfindungsgemäße Verbundwerkstoff wesentlich weniger empfindlich auf die Anwesenheit von Wasser bei erhöhten Temperaturen als dies bei den aus dem Stand der Technik bekannten Materialien der Fall ist: der erfindungsgemäße Verbundwerkstoff neigt wesentlich weniger zu hydrothermaler Alterung.

Die Herstellung des erfindungsgemäßen Verbundwerkstoffs erfolgt mittels an sich bekannter, konventioneller Keramiktechnologie. Die wesentlichen Prozessschritte sind beispielsweise:
a) Pulvermischung gemäß vorgegebener Zusammensetzung in Wasser ansetzen, ggfls. Verwendung von Verflüssigern zur Vermeidung der Sedimentation.
b) Homogenisieren im Dissolver (schnelllaufender Rührer).
c) Mahlen in Rührwerkskugelmühle, dabei Erhöhung der spezifischen Oberfläche der Pulvermischung (= Zerkleinerung).
d) Evtl. Zugabe von organischen Bindern.
e) Sprühtrocknen, dabei entsteht ein rieselfähiges Granulat mit definierten Eigenschaften.
h) Spanabhebende Grünbearbeitung, dabei wird unter Berücksichtigung der Sinterschwindung weitgehend die Endkontur abgebildet.
i) Vorbrand, dabei Schwindung auf ca. 98% der theoretischen Dichte. Die noch verbleibenden Restporen sind nach außen geschlossen.
j) Heißisostatisches Pressen unter hoher Temperatur und hohem Gasdruck, dadurch praktisch vollständige Endverdichtung.
k) So genannter Weißbrand, dadurch wird das beim heißisostatischen Pressen erzeugte Ungleichgewicht der Sauerstoffionen in der Keramik ausgeglichen.
I) Hartbearbeitung durch Schleifen und Polieren.
m) Tempern.

Verwendet werden kann der erfindungsgemäße Verbundwerkstoff beispielsweise zur Herstellung von Sinterformkörpern, zur Herstellung von Bauteilen mit der Fähigkeit zur Energieabsorption bei dynamischer Belastung in der Medizintechnik, zur Herstellung von Orthesen und Endoprothesen, beispielsweise zu Hüftgelenk- oder Kniegelenkimplantaten, Bohrern, beispielsweise für medizinische Anwendungen, Maschinenbaukomponenten, die tribologisch, chemisch und/oder thermisch beansprucht werden.

Die vorliegende Erfindung betrifft folglich einen Verbundwerkstoff aus Aluminiumoxid als keramische Matrix, darin dispergiertem Zirkonoxid und gegebenenfalls weiteren Zuschlagstoffen, wobei
➢ der Verbundwerkstoff als erste Phase einen Aluminiumoxidanteil von mindestens 65 Vol.-% und als zweite Phase einen Zirkonoxidanteil von 10 bis 35 Vol.-%, gegebenenfalls einen oder mehrere anorganische Zuschlagstoffe enthält und wobei das Zirkonoxid, bezogen auf den Geamtzirkonoxidgehalt zu 80 bis 99 %, bevorzugt zu 90 bis 99 %, in der tetragonalen Phase vorliegt und wobei der Gesamtgehalt an chemischen Stabilisatoren < 0,2 Mol% beträgt, so dass die Stabilisierung der tetragonalen Phase des Zirkonoxids zum überwiegenden Teil nicht chemisch sondern mechanisch erfolgt.

Besonders bevorzugt ist ein erfindungsgemäßer Verbundwerkstoff, bei dem
➢ die Zirkonoxidpartikel eine Korngröße von durchschnittlich 0,1 bis 0,5 µm, bevorzugt von durchschnittlich 0,15 bis 0,25 µm aufweisen;
➢ der Gehalt an chemischen Stabilisatoren relativ zum Zirkonoxid auf Werte begrenzt ist, die deutlich unterhalb der im Stand der Technik für die jeweilig verwendeten chemischen Stabilisatoren liegen;
➢ der Anteil an chemischen Stabilisatoren im erfindungsgemäßen Verbundwerkstoff (Anteil jeweils relativ zum Zirkonoxidgehalt) für Y₂O₃ ≤ 1,5 Mol%, bevorzugt ≤ 1,3 Mol%, für CeO₂ ≤ 3 Mol%, für MgO ≤ 3 Mol% und für CaO ≤ 3 Mol% beträgt;
➢ der Verbundwerkstoff keinen chemischen Stabilisator enthält;
➢ das Aluminiumoxid und/oder das Zirkonoxid lösliche Bestandteile enthält;
➢ als lösliche Bestandteile im Aluminiumoxid und/oder im Zirkonoxid ein oder mehrere der folgenden Elemente vorliegen: Cr, Fe, Mg, Ti, Y, Ce, Ca, Lanthanide und/oder V.

Weiterhin betrifft die vorliegende Erfindung die Verwendung des erfindungsgemäßen Verbundwerkstoffs
➢ zur Herstellung von Sinterformkörpern;

Weiterhin betrifft die vorliegende Erfindung die Verwendung des erfindungsgemäßen Verbundwerkstoffs
➢ zur Herstellung von Sinterformkörpern;
➢ zur Herstellung von Bauteilen mit der Fähigkeit zur Energieabsorption bei dynamischer Belastung;
➢ in der Medizintechnik;
➢ zur Herstellung von künstlichen Prothesen in der Medizintechnik, beispielsweise zur Herstellung von Orthesen und Endoprothesen;
➢ zur Herstellung von Hüftgelenk- und Kniegelenkimplantaten.

## Patentansprüche

1. Verbundwerkstoff aus Aluminiumoxid als keramische Matrix, darin dispergiertem Zirkonoxid, wobei der Verbundwerkstoff als erste Phase einen Aluminiumoxidanteil von mindestens 65 Vol.-% und als zweite Phase einen Zirkonoxidanteil von 10 bis 35 Vol.-% enthält, und wobei das Zirkonoxid, bezogen auf den Geamtzirkonoxidgehalt, zu 80 bis 99 %in der tetragonalen Phase vorliegt, **dadurch gekennzeichnet, dass** der Gesamtgehalt an chemischen Stabilisatoren < 0,2 Mol% ist, so dass die Stabilisierung der tetragonalen Phase des Zirkonoxids zum überwiegenden Teil nicht chemisch sondern mechanisch durch Einbettung in die Aluminiumoxid-Matrix erfolgt.

2. Verbundwerkstoff gemäß Anspruch 1, **dadurch gekennzeichnet, dass** das Zirkonoxid zu 90 bis 99 % in der tetragonalen Phase vorliegt.

3. Verbundwerkstoff gemäß Anspruch 1 oder 2, **dadurch gekennzeichnet, dass** die Zirkonoxidpartikel eine Korngröße von durchschnittlich 0,1 bis 0,5 µm, bevorzugt von durchschnittlich 0,15 bis 0,25 µm aufweisen.

4. Verbundwerkstoff gemäß einem oder mehreren der vorhergehenden Ansprüche, **dadurch gekennzeichnet, dass** der Anteil an chemischen Stabilisatoren im erfindungsgemäßen Verbundwerkstoff (Anteil jeweils relativ zum Zirkonoxidgehalt) für Y₂O₃ ≤ 1,5 Mol%, bevorzugt ≤ 1,3 Mol%, für CeO₂ ≤ 3 Mol%, für MgO ≤ 3 Mol% und für CaO ≤ 3 Mol% beträgt.

5. Verbundwerkstoff gemäß einem oder mehreren der vorhergehenden Ansprüche, **dadurch gekennzeichnet, dass** der Verbundwerkstoff keinen chemischen Stabilisator enthält.

6. Verbundwerkstoff gemäß einem oder mehreren der vorhergehenden Ansprüche, **dadurch gekennzeichnet, dass** das Aluminiumoxid und/oder das Zirkonoxid lösliche Bestandteile enthält.

7. Verbundwerkstoff gemäß einem oder mehreren der vorhergehenden Ansprüche, **dadurch gekennzeichnet, dass** als lösliche Bestandteile im Aluminiumoxid und/oder im Zirkonoxid ein oder mehrere der folgenden Elemente vorliegen: Cr, Fe, Mg, Ti, Y, Ce, Ca, Lanthanide und/oder V.

8. Verwendung des Verbundwerkstoffs gemäß einem oder mehreren der Ansprüche 1 bis 8 zur Herstellung von Sinterformkörpern.

9. Verwendung des Verbundwerkstoffs gemäß einem oder mehreren der Ansprüche 1 bis 7 zur Herstellung von Bauteilen mit der Fähigkeit zur Energieabsorption bei dynamischer Belastung.

10. Verwendung des Verbundwerkstoffs gemäß einem oder mehreren der Ansprüche 1 bis 7 in der Medizintechnik.

11. Verwendung des Verbundwerkstoffs gemäß einem oder mehreren der Ansprüche 1 bis 7 zur Herstellung von künstlichen Prothesen in der Medizintechnik, beispielsweise zur Herstellung von Orthesen und Endoprothesen.

12. Verwendung des Verbundwerkstoffs gemäß einem oder mehreren der Ansprüche 1 bis 7 zur Herstellung von Hüftgelenk- und Kniegelenkimplantaten.

## Claims

1. A composite material of aluminium oxide as a ceramic matrix, zirconium oxide dispersed therein, wherein the composite material contains as a first phase a proportion of aluminium oxide of at least 65% by volume and as a second phase a proportion of zirconium oxide of 10 to 35% by volume, and wherein the zirconium oxide, relative to the total zirconium-oxide content, is present to 80 to 99% in the tetragonal phase, **characterised in that** the total content of chemical stabilizers is < 0.2 mol % so that the stabilization of the tetragonal phase of the zirconium oxide for the most part does not take place chemically, but mechanically by embedding into the aluminium-oxide matrix.

2. A composite material according to claim 1, **characterised in that** the zirconium oxide is present to 90 to 99 % in the tetragonal phase.

3. A composite material according to claim 1 or 2, **characterised in that** the zirconium-oxide particles have a grain size of on average 0.1 to 0.5 µm, preferably of on average 0.15 to 0.25 µm.

4. A composite material according to one or more of the preceding claims, **characterized in that** the proportion of chemical stabilizers in the composite material in accordance with the invention (proportion in each case relative to the zirconium-oxide content) amounts for Y₂O₃ to ≤ 1.5 mol %, preferably ≤ 1.3 mol %, for CeO₂ to ≤ 3 mol %, for MgO to ≤ 3 mol % and for CaO to ≤ 3 mol %.

5. A composite material according to one or more of the preceding claims, **characterised in that** the composite material does not contain a chemical stabilizer.

6. A composite material according to one or more of the preceding claims, **characterised in that** the aluminium oxide and/or the zirconium oxide contain/contains soluble constituents.

7. A composite material according to one or more of the preceding claims, **characterised in that** one or more of the following elements is/are present as soluble constituents in the aluminium oxide and/or in the zirconium oxide: Cr, Fe, Mg, Ti, Y, Ce, Ca, lanthanides and/or V.

8. Use of the composite material according to one or more of claims 1 to 8 for the production of sintered shaped bodies.

9. Use of the composite material according to one or more of claims 1 to 7 for the production of components having the capacity to absorb energy in the event of dynamic loading.

10. Use of the composite material according to one or more of claims 1 to 7 in medical technology.

11. Use of the composite material according to one or more of claims 1 to 7 for the production of artificial prostheses in medical technology, for example for the production of orthoses and endoprostheses.

12. Use of the composite material according to one or more of claims 1 to 7 for the production of hip-joint and knee-joint implants.

## Revendications

1. Matériau composite à base d'oxyde d'aluminium, en tant que matrice de céramique, et d'oxyde de zirconium dispersé dedans, lequel matériau composite contient comme première phase de l'oxyde d'aluminium, en une proportion d'au moins 65 % en volume, et comme seconde phase de l'oxyde de zirconium, en une proportion de 10 à 35 % en volume, et dans lequel l'oxyde de zirconium, sur la base de la teneur totale en oxyde de zirconium, se présente pour 80 à 99 % dans la phase tétragonale, **caractérisé en ce que** la teneur totale en stabilisants chimiques est inférieure à 0,2 % en moles, de sorte que la stabilisation de la phase tétragonale de l'oxyde de zirconium s'effectue de manière prépondérante non pas chimiquement, mais mécaniquement, par incorporation dans la matrice d'oxyde d'aluminium.

2. Matériau composite selon la revendication 1, **caractérisé en ce que** l'oxyde de zirconium se présente pour 90 à 99 % dans la phase tétragonale.

3. Matériau composite selon la revendication 1 ou 2, **caractérisé en ce que** les particules d'oxyde de zirconium ont une taille de particules de 0,1 à 0,5 µm en moyenne, et de préférence, de 0,15 à 0,25 µm en moyenne.

4. Matériau composite selon l'une ou plusieurs des revendications précédentes, **caractérisé en ce que** les proportions des stabilisants chimiques dans le matériau composite selon l'invention (proportions respectives par rapport à la tenseur en oxyde de zirconium) s'élèvent, pour Y₂O₃, à au plus 1,5 % en moles et de préférence au plus 1,3 % en moles, pour CeO₂, à au plus 3 % en moles, pour MgO, à au plus 3 % en moles, et pour CaO, à au plus 3 % en moles.

5. Matériau composite selon l'une ou plusieurs des revendications précédentes, **caractérisé en ce que** le matériau composite ne contient aucun stabilisant chimique.

6. Matériau composite selon l'une ou plusieurs des revendications précédentes, **caractérisé en ce que** l'oxyde d'aluminium et/ou l'oxyde de zirconium continent ou contiennent des constituants solubles.

7. Matériau composite selon l'une ou plusieurs des revendications précédentes, **caractérisé en ce que**, comme constituants solubles dans l'oxyde d'aluminium et/ou l'oxyde de zirconium, un ou plusieurs des éléments suivants sont présents : Cr, Fe, Mg, Ti, Y, Ce, Ca, lanthanides et/ou V.

8. Utilisation d'un matériau composite selon l'une ou plusieurs des revendications 1 à 8 pour la fabrication de corps façonnés frittés.

9. Utilisation d'un matériau composite selon l'une ou plusieurs des revendications 1 à 7 pour la fabrication de composants ayant la capacité d'absorber de l'énergie sous le coup d'une charge dynamique.

10. Utilisation d'un matériau composite selon l'une ou plusieurs des revendications 1 à 7, dans la technologie médicale.

11. Utilisation d'un matériau composite selon l'une ou plusieurs des revendications 1 à 7 pour la fabrication de prothèses artificielles dans la technologie médicale, par exemple pour la production d'orthèses et d'endoprothèses,

12. Utilisation d'un matériau composite selon l'une ou plusieurs des revendications 1 à 7 pour la fabrication d'implants d'articulation de hanche ou d'articulation de genou.
